# EUROPEAN PATENT SPECIFICATION

(11) **EP 3 743 374 B1**
(45) Date of publication and mention of the grant of the patent: **09.03.2022**
(21) Application number: 19701111.7
(22) Date of filing: 22.01.2019
(51) Int. Cl.: C01B 25/163, C07C 51/60, C07C 407/00

(54) **PROCESS FOR THE PRODUCTION OF A FERTILIZER**
VERFAHREN ZUR HERSTELLUNG EINES DÜNGEMITTELS
PROCÉDÉ DE PRODUCTION D'UN FERTILISANT

(30) Priority: 25.01.2018 EP 18153427
(43) Date of publication of application: 02.12.2020
(73) Proprietor: Nouryon Chemicals International B.V., 6824 BM Arnhem (NL)
(72) Inventor: TAMMER, Martinus Catharinus, 7431 ZA Diepenveen (NL); VAN DEN BERG, Michel, 6661 JM Elst (NL); TER HORST, Bjorn, 3951 AE Maarn (NL)
(74) Representative: LKGlobal UK Ltd.
(86) International application number: PCT/EP2019/051512
(87) International publication number: WO 2019/145304

(56) References cited:
- EP-A1- 3 133 057
- FR-A1- 2 624 509
- US-B1- 8 585 796
- DATABASE WPI Week 201341 Thomson Scientific, London, GB; AN 2013-H54889 XP002782811, & CN 102 838 473 A (XUZHOU LVYI AGRIC TECHNOLOGY CO LTD) 26 December 2012 (2012-12-26)

## Description

The present invention relates to a process for the removal of organics from an acid chloride production waste stream. The invention also relates to the production of potassium phosphite.

Acid chlorides are conventionally produced by reacting a carboxylic acid with PCl₃ to form acid chloride and phosphorous acid (H₃PO₃). This H₃PO₃ is a waste stream that cannot be sent to a biological waste water treatment unit, as its phosphorous content is too high. It is therefore desired to use this material in another process.

Such use, however, requires that organics (e.g. acid chloride residues) are removed from the crude H₃PO₃. Unfortunately, removal of organics from crude H₃PO₃ is difficult. Even after removal of an organic layer, the H₃PO₃ remains unstable: new organic layers keep forming during storage, finally resulting in blackening of the H₃PO₃.

An object of the present invention is therefore the provision of a process that enables quick and easy removal of organics from H₃PO₃-containing waste streams.

It has now been found that this object can be achieved by reacting the crude H₃PO₃ with a potassium compound towards potassium phosphite. Organics can be easily removed from the resulting potassium phosphite solution, resulting in a clear potassium phosphite solution.

Potassium phosphite is used in the agro industry as an antifungal fertilizer. It is a fungicide and at the same time contains an important nutrient: potassium. It is non-toxic and provides both protective and curative responses against various fungal pathogens, like *Phytophtora, Rhizoctonia, Pythium,* and *Fusarium.*

US8585796B1 discloses a method of making a fertilizer composition, comprising heating phosphorous acid and potassium hydroxide in an aqueous solution at a temperature sufficient to form a polyphosphite compound effective against both fungal and bacterial plant pathogens, and allowing the solution to cool.

For its application as fungicide and/or fertilizer, potassium phosphite is generally sold in aqueous solutions of about 50 wt%, and used in strong dilution.

Transforming crude H₃PO₃ waste streams into potassium phosphite not only enables quick and easy removal of organics from the acid chloride waste stream, it also allows for the production of a valuable product from a waste stream and the production of potassium phosphite from a sustainable source.

An object of the present invention is therefore the provision of a process that enables quick and easy removal of organics from H₃PO₃-containing waste streams. Another object is the production of potassium phosphite from waste phosphorous acid. A further object is to turn waste phosphorous acid into high grade material.

It has now been found that this object can be achieved by reacting the crude H₃PO₃ with a potassium compound towards potassium phosphite. Organics can be easily removed from the resulting potassium phosphite solution, resulting in a clear potassium phosphite solution.

The present invention relates to a process for the production of a compound comprising potassium phosphite, comprising the steps of:
a. reacting carboxylic acid of the formula R-(C(=O)OH)ₙ with phosphorous trichloride (PCl₃) towards a mixture comprising phosphorous acid (H₃PO₃) and acid chloride of the formula R-(C(=O)Cl)ₙ; wherein R is a linear or branched alkyl or alkanediyl group with 1-20 carbon atoms and n is 1 or 2,
b. subjecting said mixture to a separation step, thereby obtaining (i) a fraction comprising crude phosphorous acid (H₃PO₃) and (ii) a fraction comprising acid chloride,
c. adding water and a potassium compound selected from KOH, KHCO₃ and K₂CO₃ to the fraction comprising crude phosphorous acid, thereby forming an aqueous solution comprising potassium phosphite, and
d. removing organic compounds from said aqueous solution.

The resulting aqueous solution comprises potassium phosphite. The term potassium phosphite refers to compounds with the general formula KₓH₃₋ₓPO₃ - wherein x is an average value in the range 1-2 - such as K₂HPO₃, KH₂PO₃, and combinations thereof.

Potassium phosphite can be used as a fungicide and/or fertilizer in the agro industry. The resulting acid chloride can be used for the synthesis of other organic compounds, such as organic peroxides, more specifically diacyl peroxides and peroxyesters.

### Step a

This step involves the reaction between a carboxylic acid and PCl₃.

The carboxylic acid has the formula R-(C(=O)OH)ₙ, wherein R is a linear or branched alkyl or alkanediyl group with 1-20, preferably 3-17, even more preferably 5-17, and most preferably 7-17 carbon atoms. The value of n is either 1 (resulting in a mono-acid) or 2 (resulting in a di-acid).

Examples of preferred carboxylic acids are mono-acids. Preferred monoacids are isobutanoic acid, n-butanoic acid, neopentanoic acid (pivalic acid), n-pentanoic acid (valeric acid), n-hexanoic acid, n-octanoic acid, 2-ethylhexanoic acid, 3,5,5-trimethylhexanoic acid, (neo)heptanoic acid, (neo)decanoic acid, cyclohexane carboxylic acid, and lauric acid.

The carboxylic acid functionalities and the PCl₃ react in a molar ratio 3:1, but it is preferred to use an excess of PCl₃. Preferably, a molar excess of 0-80%, more preferably of 10-50%, and most preferably of 15-40% PCl₃ is used.

The carboxylic acid functionalities and the PCl₃ are therefore preferably reacted in a molar ratio of carboxylic acid functionalities to PCl₃ of 1.5:1-3.0:1, more preferably 2.0:1-2.7:1, and most preferably 2.2:1-2.6:1. Hence, if a mono-acid is used, it is preferably reacted in a molar ratio carboxylic acid to PCl₃ of 1.5:1-3.0:1, more preferably 2.0:1-2.7:1, and most preferably 2.2:1-2.6:1. If a di-acid is used, it is preferably reacted in a molar ratio carboxylic acid to PCl₃ of 0.75:1-1.5:1, more preferably 1.0:1-1.35:1, and most preferably 1.1:1-1.3:1.

The reaction is preferably performed at a temperature in the range 20-80°C, more preferably 30-70°C, and most preferably 40-65°C.

The reaction is preferably conducted in the absence of water and organic solvents.

In a preferred embodiment, the reaction is conducted in an oxygen-free or oxygen-lean atmosphere.

The reaction results in the formation of an acid chloride and phosphorous acid.

The acid chloride has the formula R-(C(=O)Cl)ₙ, wherein R is a linear or branched alkyl group with 1-20, preferably 3-17, even more preferably 5-17, and most preferably 7-17 carbon atoms and n is either 1 or 2. If n is 1, the acid chloride is a mono-acid chloride; if n is 2, the acid chloride is a di-acid chloride. Examples of preferred acid chlorides are mono-acid chlorides. Preferred mono-acid chlorides are isobutyryl chloride, n-butyryl chloride, neopentanoyl chloride (pivaloyl cloride), n-pentanoyl chloride (valeroyl chloride), hexanoyl chloride, n-octanoyl chloride, 2-ethylhexanoyl chloride, 3,5,5-trimethylhexanoyl chloride, (neo)heptanoyl chloride, (neo)decanoyl chloride, cyclohexane carbonyl chloride, and lauroyl chloride.

By-products may be formed in this step, such as HCI, the anhydride of the carboxylic acid, and anhydrides of the carboxylic acid and phosphorous acid. HCI and any other exiting fumes can be led through a scrubber.

### Step b

The reaction product of step a) is a bi-phasic mixture comprising a H₃PO₃-containing phase (the crude phosphorous acid-comprising fraction) and an organic phase comprising the acid chloride (the acid chloride-comprising fraction). In step b), these two phases are separated. Separation can be conducted in any suitable way, e.g. by gravity or centrifugation.

The resulting separated crude phosphorous acid-comprising fraction contains phosphorous acid and organic contaminants. It may also contain a small amount of PCl₃. The total organic contaminant concentration is generally not higher than 5.0 wt%, more preferably not higher than 2.0 wt%, and most preferably not higher than 1.0 wt%.

The acid chloride can be used as a reactant towards various chemicals. Mono-acid chlorides can be used as reactants towards various esters, anhydrides, amides, and organic peroxides, in particular diacyl peroxides and peroxyesters. Di-acid chlorides can be used to produce polyesters and polyamides.

In order to make diacyl peroxides, the mono-acid chloride is reacted with hydrogen peroxide and an alkali metal salt (or a reaction product thereof) to form symmetrical diacyl peroxides. In order to prepare asymmetrical diacyl peroxides, the acid chloride can be reacted with a peroxyacid. Peroxyesters are prepared by reacting the acid chloride with an organic hydroperoxide. These processes are well known in the art.

Preferred organic peroxides to be prepared from the acid chloride resulting from the present invention are di-isobutyryl peroxide, di-n-butyryl peroxide, dineopentanoyl peroxide (di-pivaloyl peroxide), di-n-pentanoyl peroxide (divaleroyl peroxide), di-hexanoyl peroxide, di-1-octanoyl peroxide, di-2-ethylhexoyl peroxide, di-1-nonanoyl peroxide, di-3,5,5-trimethylnonanoyl peroxide, di-neodecanoyl peroxide, and di-lauroyl peroxide.

### Step c

The crude phosphorous acid-comprising fraction is then reacted with a potassium compound selected from KOH, KHCO₃ and K₂CO₃, thereby forming an aqueous KH₂PO₃ solution. KOH is the preferred potassium compound, since the use of K₂CO₃ and KHCO₃ will lead to CO₂ production.

Water, potassium compound, and the crude phosphorous acid fraction can be combined in any order, as long as the mixture is sufficiently cooled to control the resulting exothermal reaction.

Hence, water and potassium compound can be pre-mixed and the crude phosphorous acid fraction can be dosed to said mixture. Alternatively, some of the water can be added to the crude phosphorous acid fraction or *vice versa,* followed by dosing an aqueous solution of the potassium compound. Alternatively, an aqueous solution of the potassium compound can be added to the crude phosphorous acid fraction or *vice versa,* followed by dosing the remaining amount of water.

In a preferred embodiment, water and crude phosphorous acid fraction are combined at such a rate that any PCl₃ that is present in the crude phosphorous acid fraction reacts to form pure, undiluted HCI (which evaporates as a result of the exothermal reaction).

The amounts of water and potassium compound that are combined with the crude phosphorous acid fraction are preferably such that the pH of the resulting solution remains below 5, more preferably below 4.5. If the pH exceeds this value, extraction of organics in step d) will become difficult, because water soluble K-salts of the acid chlorides will be formed.

Phosphorous acid and potassium compound are preferably combined in a molar ratio of around 1:1. It is not desired to use a significant excess of one of the components. Excess of either will decrease or increase the pH of the final solution.

The product resulting from this step is an aqueous potassium phosphite solution. The potassium phosphite concentration in this solution is preferably at least 20 wt%, more preferably at least 30 wt%, and most preferably at least 40 wt%. The potassium phosphite concentration is preferably at most 70 wt%, more preferably at most 60 wt%, and most preferably at most 50 wt%.

During the process, small amounts/traces of KH₂PO₄ may be formed. The amount KH₂PO₄ in the final solution is preferably below 10 wt%, more preferably below 5 wt% and most preferably below 1 wt%, based on the weight of potassium phosphite.

During the reaction, a precipitate may be formed, consisting or organic compounds and resulting from the organic contaminants in the phosphorous acid solution.

### Step d

In this step, the organics are removed from the solution. Depending on whether the organics are in solid or liquid form, this step can be conducted by filtration, centrifugation, distillation, steam distillation, stripping with air or nitrogen, or extraction. This step can be conducted before the addition of the potassium compound (i.e. before step c) and/or after the formation of potassium phosphite (i.e. after step c).

Examples of extraction agents are C₅₋₂₀ alkanes (such as pentane, hexane, heptane, octane, nonane, decane, undecane, dodecane, tridecane, tetradecane, pentadecane, hexadecane, heptadecane, nonadecane, icosane, toluene, xylene, cyclohexane and their isomers and mixtures of such alkanes) and organic esters such as natural oils and esters of C₁₋₁₈ mono-, di-, tri-, tetra-, or poly-alcohols (preferably ethanol, propanol, butanol, glycerol) and C₂₋₂₄ mono-, di-, tri-, tetra-, or poly-acids (preferably benzoic acid, phthalic acid, 1,2-cyclohexane dicarboxylic acid, capric acid, lauric acid, myristic acid, palmitic acid, stearic acid and oleic acid).

Preferred extraction agents are materials that have food or food contact approval, such as di-isononyl-1,2-cyclohexaandicarboxylate (DINCH) and natural oils having such approval.

The resulting aqueous solution can be used - after further dilution - as a fungicide and/or fertilizer.

### EXAMPLES

### Example 1

### Steps a) and b)

Dodecanoic acid (518 g, 2.59 mol) was charged to a three-necked round bottom flask with bottom drain equipped with a mechanical overhead stirrer, a thermometer, a reflux cooler, a dropping funnel and a nitrogen purge. The reflux cooler was connected to a double wash vessel to trap HCI and PCl₃ (first flask was empty; the second one was filled with water).

Dodecanoic acid was heated to 63°C, resulting in a transparent melt. PCl₃ (100 ml, 1.15 mol) was added *via* the dropping funnel in 20 to 30 minutes; the temperature was maintained at 63°C. Stirring was stopped after the addition of the PCl₃ and the mixture was left to stand for 3 hours at 63°C. The warm crude H₃PO₃ solution (containing PCl₃ and several polyphosphorous compounds) was collected at the bottom of the flask and this phase was drained off as a slightly hazy and viscous liquid (73 g, 890 mmol, 78 % yield). The remaining crude dodecanoyl chloride (518 g, 2586 mmol, 102%) was isolated as a colorless hazy liquid and was used as such in the production of dilauroyl peroxide.

### Steps c) and d)

Water (13.32 g) and KOH (50.0 g, 45 wt%, 0.40 mol) were charged to a 100 mL beaker, equipped with a bottom drain and thermometer. The solution was stirred with a mechanical overhead stirrer at 600 rpm. The beaker was placed in a 1000mL beaker containing ice water and was cooled down to 5°C. 47 gram of the crude H₃PO₃ solution of step b) was added to the stirred solution. The dosing speed was set at such a rate that the temperature never exceeded 30°C (dosing took 20 minutes). During said dosing, a white precipitate was formed. After the dosing, stirring was stopped and the reaction mixture was cooled to 10°C. The chilled mixture was filtered under reduced pressure over a G3 glass filter. The resulting slightly hazy solution was subsequently filtered over a G4 glass filter. The second filtration yielded a clear colorless 50 wt% KH₂PO₃ solution (88.2 g, 92% yield). Total organics < 0.1 wt% (by NMR); total chloride content 0.36 wt%.

### Example 2

Steps a) and b) of Example 1 were repeated.

Water (16.5 g) was carefully added to stirred crude H₃PO₃ (38.4 g, 0.47 mol). The temperature of the mixture rose quickly, which resulted in HCI formation (the crude H₃PO₃ contained some PCl₃ residues). The vapours were removed by a nitrogen purge. When the addition was completed, the resulting hazy, hot 70 wt% H₃PO₃ solution was left to cool to room temperature. During cooling, an organic phase accumulated on the top of the solution. The phases were separated by draining off the aqueous H₃PO₃-containing phase (bottom layer).

47 gram of the so-obtained 70 wt% H₃PO₃ solution (0.40 mol H₃PO₃) was charged to a 100 mL beaker, equipped with a bottom drain and thermometer. The solution was stirred with a mechanical overhead stirrer at 1400 rpm. The beaker was placed in a 1000 mL beaker containing ice water and was cooled down to 5°C. A 45 wt% KOH solution (50 gram, 0.40 mol KOH) was added in dropwise fashion via a dropping funnel at such a rate that the temperature was kept below 25°C. The addition took 24 minutes. After this addition, stirring was stopped and a slightly hazy solution with floating solids on top was observed. The slightly hazy solution was drained off and the solids were left behind in the beaker. The obtained KH₂PO₃ solution (50 wt%, density=1.38 g/ml) was subsequently filtered over a G4 glass filter, resulting in a clear and colorless solution (93.94 gram KH₂PO₃).

## Claims

1. Process for the production of a compound comprising potassium phosphite of formula KₓH₃₋ₓPO₃ wherein x is an average value in the range 1-2
- comprising the steps of:
a. reacting a carboxylic acid of the formula R-(C(=O)OH)ₙ with phosphorous trichloride (PCl₃) towards a mixture comprising phosphorous acid (H₃PO₃) and an acid chloride of the formula R-(C(=O)Cl)ₙ; wherein R is a linear or branched alkyl or alkanediyl group with 1-20 carbon atoms and n is 1 or 2,
b. subjecting said mixture to a separation step, thereby obtaining (i) a fraction comprising crude phosphorous acid (H₃PO₃) and (ii) a fraction comprising the acid chloride,
c. combining water, a potassium compound selected from KOH, KHCO₃ and K₂CO₃, and the fraction comprising crude phosphorous acid, thereby forming an aqueous solution comprising potassium phosphite, and
d. removing organic compounds from said aqueous solution.

2. Process according to claim 1 wherein in step c) the fraction comprising crude phosphorous acid is dosed to an aqueous solution of the potassium compound.

3. Process according to claim 1 or 2 wherein step d) is performed by centrifugation, filtration, extraction, distillation, steam distillation, or stripping with air or nitrogen.

4. Process according to any one of the preceding claims wherein R is a linear or branched alkyl or alkanediyl group with 3-17 carbon atoms, preferably 7-17 carbon atoms.

5. Process according to claim 4 wherein the carboxylic acid is selected from the group consisting of isobutanoic acid, n-butanoic acid, lauric acid, 3,5,5-trimethylhexanoic acid, pivaloic acid, valeric acid, n-hexanoic acid, n-octanoic acid, 2-ethylhexanoic acid, (neo)decanoic acid, (neo)heptanoic acid, cyclohexane carboxylic acid, and mixtures thereof.

6. Process according to any one of the preceding claims wherein the potassium compound is KOH.

7. Process according to any one of the preceding claims wherein n is 1.

8. Process according to claim 7 containing the additional step of reacting the acid chloride with hydrogen peroxide and an alkali metal salt to form a diacyl peroxide.

9. Process according to claim 7 containing the additional step of reacting the acid chloride with a peroxyacid and an alkali metal salt to form a diacyl peroxide.

10. Process according to claim 9 wherein the peroxy acid is selected from peracetic acid, perpropionic acid, perlauric acid, and mixtures thereof.

11. Process according to claim 7 containing the additional step of reacting the acid chloride with an organic hydroperoxide and an alkali metal salt to form a peroxyester.

12. Process according to claim 11 wherein the organic hydroperoxide is selected from the group consisting of tert-butyl hydroperoxide, tert-amyl hydroperoxide, cumyl hydroperoxide, 2,2,4,4-tetramethylbutyl hydroperoxide, 1,3-bis(2-hydroperoxypropan-2-yl)benzene, 1,4-bis(2-hydroperoxypropan-2-yl)benzene, 2,5-dihydroperoxy-2,5-dimethylhexane, 2,5-dihydroperoxy-2,5-dimethylhex-3-yne, and mixtures thereof.

## Patentansprüche

1. Verfahren zur Herstellung einer Verbindung, die Kaliumphosphit der Formel KₓH₃₋ₓPO₃ umfasst, wobei x ein durchschnittlicher Wert im Bereich von 1-2 ist,
- umfassend die Schritte des:
a. Reagierens einer Carbonsäure der Formel R-(C(=O)OH)ₙ mit Phosphortrichlorid (PCl₃) zu einer Mischung, die phosphorige Säure (H₃PO₃) und ein Säurechlorid der Formel R-(C(=O)Cl)ₙ umfasst; wobei R eine lineare oder verzweigte Alkyl- oder Alkandiylgruppe mit 1-20 Kohlenstoffatomen ist und n 1 oder 2 beträgt,
b. Unterwerfens der Mischung einem Trennungsschritt, wodurch (i) ein Anteil, der rohe phosphorige Säure (H₃PO₃) umfasst und (ii) ein Anteil, der das Säurechlorid umfasst, erhalten wird,
c. Kombinierens von Wasser, einer Kaliumverbindung ausgewählt unter KOH, KHCO₃ und K₂CO₃ und des Anteils, der rohe phosphorige Säure umfasst, wodurch eine wässrige Lösung gebildet wird, die Kaliumphosphit umfasst, und
d. Entfernens von organischen Verbindungen aus der wässrigen Lösung.

2. Verfahren nach Anspruch 1, wobei, in Schritt c), der Anteil, der rohe phosphorige Säure umfasst, auf eine wässrige Lösung der Kaliumverbindung dosiert wird.

3. Verfahren nach Anspruch 1 oder 2, wobei der Schritt d) durch Zentrifugieren, Filtrieren, Extraktion, Destillation, Dampfdestillation oder Abtreiben mit Luft oder Stickstoff ausgeführt wird.

4. Verfahren nach einem der vorhergehenden Ansprüche, wobei R eine lineare oder verzweigte Alkyl- oder Alkandiylgruppe mit 3-17 Kohlenstoffatomen, bevorzugt 7-17 Kohlenstoffatomen, ist.

5. Verfahren nach Anspruch 4, wobei die Carbonsäure aus der Gruppe ausgewählt wird bestehend aus Isobutansäure, n-Butansäure, Laurinsäure, 3,5,5-Trimethylhexansäure, Pivalonsäure, Valeriansäure, n-Hexansäure, n-Octansäure, 2-Ethylhexansäure, (Neo)decansäure, (Neo)heptansäure, Cyclohexancarbonsäure und Mischungen davon.

6. Verfahren nach einem der vorhergehenden Ansprüche, wobei die Kaliumverbindung KOH ist.

7. Verfahren nach einem der vorhergehenden Ansprüche, wobei n 1 beträgt.

8. Verfahren nach Anspruch 7, enthaltend den zusätzlichen Schritt des Reagierens des Säurechlorids mit Wasserstoffperoxid und einem Alkalimetallsalz, um ein Diacylperoxid zu bilden.

9. Verfahren nach Anspruch 7, enthaltend den zusätzlichen Schritt des Reagierens des Säurechlorids mit einer Peroxysäure und einem Alkalimetallsalz, um ein Diacylperoxid zu bilden.

10. Verfahren nach Anspruch 9, wobei die Peroxysäure unter Peressigsäure, Perpropionsäure, Perlaurinsäure und Mischungen davon ausgewählt wird.

11. Verfahren nach Anspruch 7, enthaltend den zusätzlichen Schritt des Reagierens des Säurechlorids mit einem organischen Hydroperoxid und einem Alkalimetallsalz, um einen Peroxyester zu bilden.

12. Verfahren nach Anspruch 11, wobei das organische Hydroperoxid aus der Gruppe ausgewählt wird bestehend aus tert-Butylhydroperoxid, tert-Amylhydroperoxid, Cumylhydroperoxid, 2,2,4,4-Tetramethylbutylhydroperoxid, 1,3-Bis(2-hydroperoxypropan-2-yl)benzol, 1,4-Bis(2-hydroperoxypropan-2-yl)benzol, 2,5-Dihydroperoxy-2,5-dimethylhexan, 2,5-Dihydroperoxy-2,5-dimethylhex-3-yn und Mischungen davon.

## Revendications

1. Procédé de production d'un composé comprenant du phosphite de potassium de formule KₓH₃₋ₓPO₃ dans lequel x est une valeur moyenne comprise dans la plage allant de 1 à 2
comprenant les étapes de :
a. mise en réaction d'un acide carboxylique de formule R-(C(=O)OH)ₙ avec du trichlorure phosphoreux (PCl₃) envers un mélange comprenant de l'acide phosphoreux (H₃PO₃) et un chlorure d'acide de formule R-(C(=O)Cl)ₙ ; dans lequel R est un groupe alkyle ou alcanediyle linéaire ou ramifié comprenant de 1 à 20 atomes de carbone et n vaut 1 ou 2,
b. soumission dudit mélange à une étape de séparation, obtenant ainsi (i) une fraction comprenant l'acide phosphoreux (H₃PO₃) brut et (ii) une fraction comprenant le chlorure d'acide,
c. combinaison d'eau, d'un composé de potassium sélectionné parmi KOH, KHCO₃ et K₂CO₃, et de la fraction comprenant l'acide phosphoreux brut, formant ainsi une solution aqueuse comprenant le phosphite de potassium, et
d. élimination des composés organiques de ladite solution aqueuse.

2. Procédé selon la revendication 1 dans lequel dans l'étape c), la fraction comprenant l'acide phosphoreux brut est dosée par rapport à une solution aqueuse du composé de potassium.

3. Procédé selon la revendication 1 ou 2 dans lequel l'étape d) est réalisée par centrifugation, filtration, extraction, distillation, distillation à la vapeur ou strippage à l'air ou à l'azote.

4. Procédé selon l'une quelconque des revendications précédentes dans lequel R est un groupe alkyle ou alcanediyle linéaire ou ramifié comprenant de 3 à 17 atomes de carbone, de préférence de 7 à 17 atomes de carbone.

5. Procédé selon la revendication 4 dans lequel l'acide carboxylique est sélectionné dans le groupe constitué par l'acide isobutanoïque, l'acide n-butanoïque, l'acide laurique, l'acide 3,5,5-triméthylhexanoïque, l'acide pivaloïque, l'acide valérique, l'acide n-hexanoïque, l'acide n-octanoïque, l'acide 2-éthylhexanoïque, l'acide (néo)décanoïque, l'acide (néo)heptanoïque, l'acide cyclohexanecarboxylique et des mélanges de ceux-ci.

6. Procédé selon l'une quelconque des revendications précédentes dans lequel le composé de potassium est KOH.

7. Procédé selon l'une quelconque des revendications précédentes dans lequel n vaut 1.

8. Procédé selon la revendication 7 contenant l'étape supplémentaire de mise en réaction du chlorure d'acide avec du peroxyde d'hydrogène et un sel de métal alcalin pour former un peroxyde de diacyle.

9. Procédé selon la revendication 7 contenant l'étape supplémentaire de mise en réaction du chlorure d'acide avec un peroxyacide et un sel de métal alcalin pour former un peroxyde de diacyle.

10. Procédé selon la revendication 9 dans lequel le peroxyacide est sélectionné parmi l'acide peracétique, l'acide perproprionique, l'acide perlaurique et des mélanges de ceux-ci.

11. Procédé selon la revendication 7 contenant l'étape supplémentaire de mise en réaction du chlorure d'acide avec un hydroperoxyde organique et un sel de métal alcalin pour former un peroxyester.

12. Procédé selon la revendication 11 dans lequel l'hydroperoxyde organique est sélectionné dans le groupe constitué par l'hydroperoxyde de tert-butyle, l'hydroperoxyde de tert-amyle, l'hydroperoxyde de cumyle, l'hydroperoxyde de 2,2,4,4-tétraméthylbutyle, le 1,3-bis(2-hydroperoxypropan-2-yl)benzène, le 1,4-bis(2-hydroperoxypropan-2-yl)benzène, le 2,5-dihydroperoxy-2,5-diméthylhexane, le 2,5-dihydroperoxy-2,5-diméthylhex-3-yne et des mélanges de ceux-ci.
